# EUROPÄISCHE PATENTSCHRIFT

(11) **EP 3 435 854 B1**
(45) Veröffentlichungstag und Bekanntmachung des Hinweises auf die Patenterteilung: **28.04.2021**
(21) Anmeldenummer: 17716811.9
(22) Anmeldetag: 30.03.2017
(51) Int. Cl.: A61B 5/00

(54) **VORRICHTUNG UND VERFAHREN ZUR POSITIONSERFASSUNG EINES MOBILEN MEDIZINISCHEN GERÄTES**
APPARATUS AND METHOD FOR DETECTING THE POSITION OF A MOBILE MEDICAL DEVICE
PROCÉDÉ ET DISPOSITIF DE DÉTECTION DE LA POSITION D'UN DISPOSITIF MÉDICAL MOBILE

(30) Priorität: 30.03.2016 DE 102016105793
(43) Veröffentlichungstag der Anmeldung: 06.02.2019
(73) Patentinhaber: Piur Imaging GmbH, 1030 Wien (AT)
(72) Erfinder: BAUER, Robert, 82008 Unterhaching (DE); SPRUNG, Julian, 80337 München (DE)
(74) Vertreter: Zimmermann & Partner Patentanwälte mbB
(86) Internationale Anmeldenummer: PCT/EP2017/057559
(87) Internationale Veröffentlichungsnummer: WO 2017/167887

(56) Entgegenhaltungen:
- WO-A1-2010/130723
- WO-A1-2017/048430
- GB-A- 2 510 452
- US-A1- 2005 203 394
- US-A1- 2007 027 395
- US-A1- 2015 094 605

## Beschreibung

Aspekte der Erfindung beziehen sich auf Vorrichtung zum Erfassen der Position eines mobilen medizinischen Gerätes an der Hautoberfläche eines Patienten. Insbesondere erfolgt die Positionserfassung mittels eines von einer Signalquelle am Körper des Patienten abgegebenen und entlang der Hautoberfläche propagierenden elektrischen Signals. Das elektrische Signal ist ein eigens von einer hierfür vorgesehenen Spannungsquelle erzeugtes Signal.

### Technischer Hintergrund

In verschiedenen Anwendungsgebieten kann es in der Medizin nützlich sein, die Position mobiler medizinischer Geräte - etwa von medizinischen Instrumenten oder von Kameras oder Detektoren für die medizinische Bildgebung - zu bestimmen. Diese Positionsbestimmung wird auch als Tracking bezeichnet.

Beispiele für bekannte Verfahren für die Positionsbestimmung sind Infrarot-Stereokameratracking mit aktiven Lichtquellen oder Reflektionsmarkern, Elektromagnetisches Tracking mit Feldgenerator und Induktionsspulen, und Markertracking mittels optischer Kameras (wobei als Marker z.B. Schachbrettmuster oder QR-Codes eingesetzt werden). Derartige Positionsbestimmungen sind beispielsweise bei bildgebenden Verfahren von Vorteil, wie in der WO 2008/142172 A2 beschrieben.

Diese und ähnliche bekannte Verfahren sind allerdings zumeist relativ aufwändig und teurer oder weisen andere Limitierungen bei der Bedienung auf, wie beispielsweise ein stark begrenztes Messvolumen (EM-Tracking), teils Unverträglichkeit mit Herzschrittmachern und Störungen durch (ferromagnetische) Metalle im Messbereich (EM-Tracking), die Notwendigkeit von Sichtkontakt (Kamerabasiertes Tracking) und damit einhergehende Einschränkungen der Ergonomie. Zusätzlich erfordern sie eine aufwändige Installation und Kalibrierung, und bieten nur eine eingeschränkte Flexibilität.

US 5,795,298 beschreibt eine gemeinsame Hardware für EKG-Messung und Tracking. Das Tracking erfolgt hierbei auf vorbekannte Weise mittels elektromagnetischen Feldern im Raum

Eine weitere Vorrichtung zur Positionserfassung wird in GB 2 510 452 beschrieben.

### Zusammenfassung der Erfindung

Vor diesem Hintergrund wird eine Vorrichtung zum Erfassen der Position eines mobilen medizinischen Gerätes gemäß Anspruch 1 vorgeschlagen.

### Ausführliche Beschreibung der Figuren

Weitere Ausführungsformen und mögliche Aspekte der Erfindung ergeben sich aus den abhängigen Ansprüchen, der Beschreibung und den Figuren. Darin zeigen:
Fig. 1 eine schematische Ansicht einer Ausführungsform zur passiven Positionsbestimmung mittels eines EKG-Signals vom Herzen eines Patienten;
Fig. 2 eine schematische Ansicht einer Ausführungsform zur aktiven Positionsbestimmung mit einer Empfangselektrode am mobilen medizinischen Gerät;
Fig. 3 eine schematische Ansicht einer Ausführungsform zur aktiven Positionsbestimmung mit einer Sendeelektrode am mobilen medizinischen Gerät;
Fig. 4 Verfahrensschritte eines Verfahrens zum Erfassen der Position eines mobilen medizinischen Gerätes gemäß einem Aspekt der Erfindung;
Fig. 5 eine schematische Darstellung einer Ausführungsform zur aktiven Positionsbestimmung mit einer Empfangselektrode am mobilen medizinischen Gerät; und
Fig. 6 schematische Darstellung einer weiteren Ausführungsform zur aktiven Positionsbestimmung mit einer Empfangselektrode am mobilen medizinischen Gerät und einer von der Sendeelektrode verschiedenen Referenzelektrode.

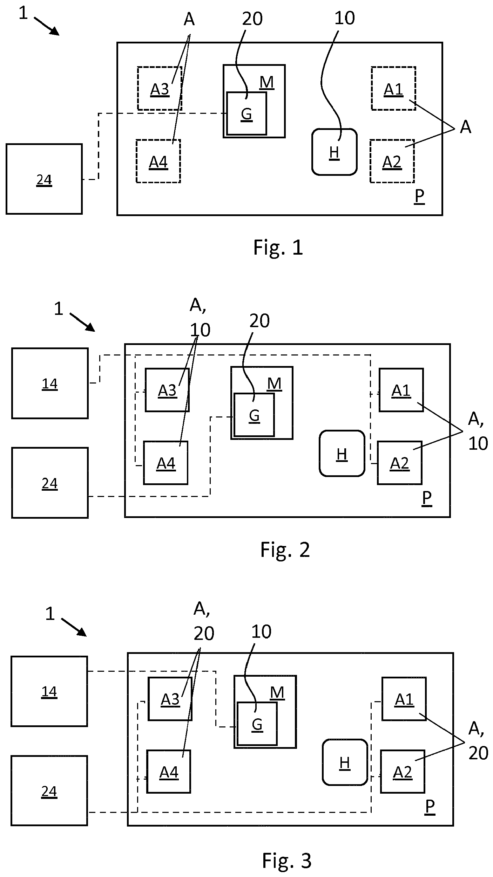

Mit Bezug auf Fig. 1 wird zunächst ein erstes Beispiel einer Vorrichtung 1, obzwar für sich genommen nicht Teil der Erfindung, zum besseren Verständnis der Erfindung beschrieben. Die Vorrichtung 1 ist für die passive Positionsbestimmung eines mobilen medizinischen Gerätes M mittels eines EKG-Signals vom Herzen H eines Patienten P eingerichtet. Hierin werden auch Herzzellen wie Schrittmacherzellen und dergleichen zum Herzen gezählt. Als EKG-Signal wird hierin ein durch die elektrische Herzaktivität generiertes elektrisches Signal betrachtet. Eine Visualisierung des EKG-Signals ist nicht zwingend erforderlich.

Zu diesem Zweck ist eine Geräteelektrode G an dem mobilen medizinischen Gerät M angebracht. Das medizinische Gerät ist ein Ultraschall-Gerät, kann aber auch ein anderes Gerät sein, das bei Benutzung an der Hautoberfläche des Patienten zu positionieren ist. Die Geräteelektrode G ist derart an dem mobilen medizinischen Gerät M angebracht, dass die Geräteelektrode G bei Benutzung (bei bestimmungsgemäßer Positionierung an der Hautoberfläche des Patienten) des mobilen medizinischen Geräts M einen elektrischen Kontakt mit der Hautoberfläche des Patienten P herstellt. Die Geräteelektrode G dient als Empfangselektrode 20 zum Empfangen des EKG-Signals, wenn der Kontakt zwischen der Geräteelektrode G und der Hautoberfläche des Patienten P hergestellt ist.

Hierbei ist für einen (elektrischen) Kontakt nicht zwingend ein direkter bzw. galvanischer Kontakt erforderlich. Auch eine induktive oder kapazitive Kopplung einer Elektrode an die Hautoberfläche des Patienten wird hierin als (elektrischer) Kontakt verstanden. Mit der Empfangselektrode 20 ist eine Auswerteeinheit 24 mittels einer Verbindungsleitung operativ verbunden, wie durch die gestrichelte Linie in Fig. 1 schematisch dargestellt ist. Die Verbindungsleitung kann ein Signalkabel sein, oder eine drahtlose Verbindung über einen Sender und Empfänger für das EKG-Signal herstellen.

Die Auswerteeinheit 24 empfängt das von der Empfangselektrode 20 empfangene EKG-Signal. Dieses Signal kann zuvor über zwischengelagerte Signalverarbeiter (nicht dargestellt) verstärkt, gefiltert und/oder auf eine andere Weise prozessiert werden. Die Auswerteeinheit 24 wertet sodann das empfangene EKG-Signal aus, um anhand einer Charakteristik des Signals eine Ortsinformation der Empfangselektrode 20 relativ zum Herzen H des Patienten P zu errechnen.

Im Folgenden werden vorteilhafte Aspekte für die Berechnung der Ortsinformation beschrieben. Die Berechnung der Ortsinformation gemäß dieser Aspekte basiert auf der folgenden Erkenntnis: Das menschliche Herz erzeugt elektrische Aktivität mit einem charakteristischen elektrischen Feld. In einfacher Näherung kann die Auswerteeinheit das Feld etwa als das Feld eines elektrischen Dipols mit wechselnder Richtung und Betrag des Dipolmomentes in schwach leitender Umgebung modellieren. Durch dieses Feld lassen sich auf der Haut zeitlich ändernde Körperoberflächenpotentiale messen. Diese werden in der regulären EKG-Technik bereits an verschiedenen Punkten abgeleitet, verstärkt und visualisiert, um die Herzaktivität zu messen.

Das Verfahren gemäß dieser Ausführungsform nutzt dieses vom Herzen H erzeugte elektrische Feld zur Positionsbestimmung des Geräts M auf der Hautoberfläche des Patienten. Hierzu wird das mittels der Geräteelektrode G (Empfangselektrode 20) gemessene elektrische Feld mittels der Auswerteeinheit 24 im Hinblick auf die charakteristische Richtungs- und Entfernungsabhängigkeit des vom Herzen H erzeugten elektrischen Feldes ausgewertet, um eine Position der Geräteelektrode G, und somit des Geräts M, auf der Hautoberfläche des Patienten zu ermitteln.

Für die Auswertung bzw. Positionsbestimmung sind im Einzelnen verschiedene Umsetzungen möglich. In einer ersten möglichen Umsetzung kann die Vorrichtung beispielsweise eine geometrische Anordnung einer Mehrzahl von Referenzelektroden (EKG-Elektroden) umfassen; diese Referenzelektroden werden an vorbestimmten Position am Patientenkörper P verteilt angeordnet, und deren Spannungsverläufe gemessen. Das Resultat einer solchen Messung sind für jede der Referenzelektroden charakteristische Spannungsverläufe.

Eine Ausgestaltung ist beispielsweise ein zweidimensionales Gitter von eng beabstandeten Referenzelektroden, das an dem Patientenkörper bzw. einer Region desselben (etwa den Torso) verteilt ist. Der Gitterabstand kann beispielsweise weniger als 20 cm, bevorzugt 1 cm - 10 cm, betragen. Die Anzahl der Referenzelektroden kann mindestens 16, mindestens 25 oder sogar mindestens 36 betragen.

In einer weiteren Ausgestaltung können die Referenzelektroden als (EKG-)Ableitungselektroden A in üblicher Anordnung an verschiedenen Positionen des Körpers des Patienten P angebracht sein (in Fig. 1 gestrichelt als vier Ableitungselektroden A1, A2, A3, A4 dargestellt). Die Anzahl von Referenzelektroden ist beliebig, z.B. 1, 4, 9 oder sogar mehr Referenzelektroden. Jeder der Referenzelektroden A, oder Gruppe von Referenzelektroden, kann ein jeweiliger EKG-Kanal zugeordnet sein. Die Ableitungselektroden können beliebige, vorzugsweise galvanisch an den Körper gekoppelte EKG-Ableitungselektroden sein, z.B. die von der Firma Kendall vertriebenen Modelle H124SG oder H92SG.

Eine mögliche Anordnung von Referenzelektroden ist wie folgt: Mehrere (etwa 9) Referenzelektroden sind derart am Körper des Patienten P angeordnet, dass jeweilige Gruppen (z.B. Dreierpaare) der Referenzelektroden existieren, welche zueinander näherungsweise orthogonale Ebenen aufspannen (also Ebenen, die mit einer Toleranz von weniger als 30° zueinander orthogonal sind).

Die Auswerteeinheit 24 ist operativ über (in Fig. 1 nicht dargestellte) Verbindungsleitungen mit den Referenzelektroden A verbunden, und Ortsinformationen (etwa Richtungs- bzw. Kanalinformationen und optional Entfernungsinformationen) dieser Referenzelektroden A sind in der Auswerteeinheit 24 gespeichert.

Der an der Geräteelektrode G gemessene Spannungsverlauf (elektrisches Signal) erlaubt es nun, die Position der Geräteelektrode G durch Vergleichen des dort gemessenen Spannungsverlaufes mit allen Verläufen an den Referenzelektroden A zu bestimmen.

Beispielsweise kann die Auswerteeinheit 24 den jeweilige Übereinstimmungsgrad des EKG-Signals der Empfangselektrode 20 mit jedem der Signale der Referenzelektroden ermitteln werden, und sodann eine Position der Empfangselektrode als Funktion der Übereinstimmungsgrade errechnen. Das Berechnen der Übereinstimmungsgrade kann mittels einer Funktion erfolgen, welche die Ähnlichkeit zwischen dem an der Geräteelektrode G und dem an einer jeweiligen Referenzelektrode gemessenen Signal ausdrückt, insbesondere eine Abweichungsfunktion wie im Folgenden beschrieben. Als Signal kann hierbei der gemessene Spannungsverlauf selbst, oder eine aus dem gemessenen Spannungsverlauf abgeleitete Größe, z.B. mittels einer Normierung oder Extraktion von für den Spannungsverlauf charakteristischen Werten, angesehen werden.

Das Signal (Spannungsverlauf) ist bevorzugt ein komplett aufgezeichneter Herzzyklus (z.B. eins oder mehrere von P-Welle, QRS-Komplex und T-Welle) der Empfangselektrode 20, oder ein Teil davon. Für die Abweichungsfunktion werden bevorzugt auch transformierte Signale wie ein skalierter und/oder invertierter Spannungsverlauf einbezogen, so dass die Abweichungsfunktion die kleinste Abweichung von einem derart skalierten Signal ausgibt.

Die Abweichungsfunktion |...| kann beispielsweise eine L2-Norm (Sum of squared errors), eine Normalized Cross Correlation oder dergleichen beinhalten. Optional kann die Abweichungsfunktion eine vorherige Anpassung der Signale beinhalten, etwa eine Normierung, Projektion oder sonstige Transformation.

Die Position der Geräteelektrode G kann als die Position der Referenzelektrode mit der (gemäß einer solchen Abweichung) geringsten Abweichung ermittelt werden, etwa mittels eines Minimierungsverfahrens. Optional kann eine solche Minimierung unter Randbedingungen durchgeführt werden, etwa unter der Bedingung, dass die Position auf der Körperoberfläche liegen muss, in einer bestimmten Region (der Körperoberfläche) liegen muss oder eine andere Plausibilitätsbedingung erfüllen muss. Als Erfolgskriterium, etwa bei einem iterativen Algorithmus, kann vorgegeben werden, dass die Abweichung eine vorgegebene Grenze unterschreiten muss und/oder dass die Änderung der Abweichung eine vorgegebene Grenze unterschreiten muss.

Weitere alternative Verfahren erlauben die Positionsbestimmung mit einer geringeren Anzahl von Referenzelektroden (etwa mit den bereits genannten Ableitungselektroden A) und/oder mit einer erhöhten Genauigkeit.

Gemäß einer Ausführungsform kann die Position der Geräteelektrode durch einen Mittelwert der Positionen der Referenzelektroden mit den geringsten Abweichungen ermittelt werden (etwa aller Referenzelektroden, deren Abweichung eine vorgegebene Grenze unterschreitet, und / oder eine vorgegebene Anzahl von Referenzelektroden mit den geringsten Abweichungen). Der Mittelwert kann ein gewichteter Mittelwert sein. Die Position kann etwa als gewichteter Mittelwert der Positionen aller oder ausgewählter Referenzelektroden errechnet werden, wobei die Gewichtung bzw. Auswahl in Abhängigkeit vom ermittelten Übereinstimmungsgrad für die jeweilige der Referenzelektroden festgelegt wird (mit höherer Gewichtung bzw. Auswahl bei höherer Übereinstimmung).

Gemäß einer weiteren Variante kann die Auswerteeinheit 24 auch virtuelle Referenzsignale (z.B. EKG-Kanäle) weiterer Referenzelektroden aus den von den vorhandenen Referenzelektroden A empfangenen Referenzsignalen errechnen, auch wenn für die weiteren Referenzsignale nicht zwingend eine Referenzelektrode (EKG-Kanalelektrode) vorhanden ist. Die virtuellen Referenzsignale kann die Auswerteeinheit 24 sodann analog berücksichtigen wie oben für die gemessenen Referenzsignale beschrieben. Die Berechnung der virtuellen Referenzsignale kann beispielsweise durch Interpolation zwischen zwei oder mehr gemessenen Signalen bewerkstelligt werden. Die Interpolation kann durch eine (gewichtete) Mittelwertbildung und/oder durch das elektrische Zusammenschalten mehrerer (realer) Referenzelektroden erfolgen, optional mit geeigneten Widerständen, um das virtuelle Referenzsignal (Signal einer virtuellen Elektrode) zwischen den Referenzelektroden zu erzeugen.

Alternativ oder zusätzlich kann die Berechnung des virtuellen Referenzsignals unter Benutzung eines Modells für das vom Herzen abgestrahlte EKG-Signal erfolgen, welches ein richtungsabhängiges EKG-Modellsignal als Funktion einer Mehrzahl freier Parameter angibt: Die freien Parameter des Modells werden in dieser Variante (etwa unter Verwendung eines best-fit-Algorithmus) aus den gemessenen Referenzsignalen (z.B. EKG-Kanalsignalen) ermittelt. Das Modell kann gemäß einem oder mehrerer der hierin genannten Kriterien implementiert sein.

Gemäß einer weiteren Alternative kann auf einige oder alle der EKG-Referenzelektroden A auch verzichtet werden. In diesem Fall kann das an der Geräteelektrode gemessene Signal auch unter Berücksichtigung eines Modells für das durch das Herz erzeugte Spannungssignal am Körper des Patienten P ausgewertet werden, um eine Ortsinformation relativ zum Herzen H zu errechnen. Das Modell beinhaltet vorzugsweise eine Funktion SM(x), welche ein modelliertes Spannungssignal SM als Funktion einer Ortsvariable x auf der Hautoberfläche des Patienten P ausgibt. Die Ortsinformation kann dann als diejenige Ortsvariable x_{G} ermittelt werden, für welche das modellierte Spannungssignal SM(x_{G}) das tatsächlich an der Geräteelektrode gemessene Spannungssignal S_{G} nach einem vorgegebenen Kriterium am besten annähert. Beispielsweise kann x_{G} durch Anwenden eines Minimisierungsalgorithmus auf die Abweichungsfunktion |SM(x) - S_{G}| mit x als Minimisierungsvariable errechnet werden.

Die Ortsvariable x kann also beispielsweise einen Winkel ϕ, eine Entfernung r, oder beides (r,cp) gegenüber dem Herzen H als der Signalquelle umfassen. Der Winkel ϕ kann ein (2-dimensionaler) Raumwinkel sein. Wenn die zu ermittelnde Position von vorneherein auf die Körperoberfläche und/oder auf eine bestimmte Region des Patienten beschränkt ist, kann ein einfacher Winkel (1-dimensionale Winkelangabe innerhalb einer Ebene bzw. Mannigfaltigkeit) genügen. Optional kann die Ortsvariable x zusätzlich eine diskrete Angabe über die Regionsangabe (z.B. Ober-/Unterseite des Torso) enthalten.

Die Abweichungsfunktion |...| kann hier insbesondere eine derartige Anpassung der Signale beinhalten, die eine Abhängigkeit von der Entfernung r eliminiert bzw. verringert, etwa eine Skalierung bzw. Normierung des Signals. Eine solche Normierung kann insbesondere zur Ermittlung des Winkels ϕ als der Ortsvariable nützlich sein.

Das Modell bzw. das modellierte Spannungssignal SM(x) kann von weiteren Messgrößen abhängen. Insbesondere kann es von gemessenen elektrischen Signalen an einer oder mehreren Ableitungselektroden A abhängen.

Elemente eines solchen Modells können eins oder mehrere der folgenden sein:
a) das vom Herzen ausgesendete elektrische Signal, oder eine Hauptkomponente davon, wird als elektrische Dipolstrahlung angenähert, etwa als die Dipolstrahlung mit der geringsten Abweichung gegenüber den durch die Ableitungselektroden A gemessenen Signalen;
b) die Richtung eines Hauptvektors des elektrischen Signals variiert während eines Herzzyklus;
c) die zwischen Elektroden gemessenen Spannungen (Potentialdifferenzen) sind proportional zu einer Projektion des Dipolmomentes auf die Strecke zwischen den Elektroden;
d) das elektrische Signal fällt mit der Entfernung vom Herzen gemäß einer vorbestimmten Abstandsfunktion ab (z.B. quadratisch, aber auch detailliertere und ggf. richtungsabhängige Funktionen sind möglich); dies erlaubt die Ermittlung eines Abstands der Elektrode vom Herzen. Beispielsweise kann die Abstandsfunktion anhand einer Karte mit (durchschnittlichen oder individuell kalibrierten) Körperoberflächenpotentialen bzw. Widerständen / Impedanzen errechnet sein.
e) Das Modell kann auch inhomogene (zum Teil auch zeitlich variierende, z.B. durch Ein- und Ausatmen) elektrische Eigenschaften der verschiedenen Organe des menschlichen Körpers berücksichtigen; dies kann etwa durch eine Zeitabhängigkeit der in Punkt d) genannten Parameter erreicht werden.
   Die Auswerteeinheit kann eine Funktion beinhalten, um eine derartige periodische Zeitabhängigkeit zu erkennen und zu kompensieren, beispielsweise indem zusätzlich zu den genannten Parametern auch eine oder mehrere periodische Abweichungen für die parametrisierten Messwerte im Modell angegeben werden. Eine solche periodische Abweichung kann etwa durch eine Amplitude, Frequenz, und/oder Phase der Abweichung beschrieben und im Modell berücksichtigt sein. Die Abweichung (z.B. Frequenz und/oder Phase) kann etwa durch eine zusätzliche Sensorik (z.B. Sensor auf Torso für Atemstatus) ermittelt werden.
   Alternativ oder zusätzlich kann die Auswerteeinheit auch ein Filtermodul aufweisen, um selektiv nur Messwerte zur weiteren Verwendung zu filtern, welche einer bestimmten Phase im Atemzyklus zugeordnet sind. Das Filtermodul kann ausgestattet sein, um die Phase aus periodischen Schwankungen in den gemessenen EKG-Daten zu ermitteln, und/oder an eine zusätzliche Sensorik (z.B. Sensor auf Torso für Atemstatus) zur Ermittlung der Phase anschließbar bzw. angeschlossen sein.
f) Das Modell kann Kalibrierungsdaten nutzen, die während einem Kalibrationsvorgang vor Beginn und/oder während der Messungen erhalten worden sind, bei dem verschiedene elektrische Parameter eines Körperersatzmodells geschätzt werden (z.B. Widerstand des Armes, Hautwiderstand, etc). Die Schätzung kann z.B. durch Einprägung einer Spannung zwischen zwei Elektroden und Messung der Spannung an allen anderen mit anschließendem fitten einer Modellgleichung errechnet werden. (Hierunter wird auch das Einprägen eines Stroms oder einer anderen Regelungsgröße, die zu einer Einprägung einer Spannung führt, verstanden).
g) Zusätzlich oder alternativ kann das Modell auch auf empirisch gesammelte Durchschnittswerte zurückgreifen. Diese Durchschnittswerte sind gemäß diesem Aspekt beispielsweise in einem Speicherabschnitt des PCs hinterlegt. Die Auswerteeinheit 24 kann dann die Richtung ausgeben, die dem Durchschnittswert mit der geringsten Abweichung zugeordnet ist. Optional können auch genauere Richtungen durch (ggf. gewichtete) Interpolation zwischen mehreren Richtungen, deren Note einem gewissen Kriterium entspricht.

Wie bereits oben geschildert, kann die zu berechnende Ortsinformation eine Richtungs- und eine Entfernungsinformation relativ zum Herzen H des Patienten P umfassen. Gemäß einer bevorzugten Ausführungsform für die Berechnung der Richtungsinformation ist es nützlich, dass die Form des vom Herzen H abgegebenen EKG-Signal eine charakteristische Richtungsabhängigkeit aufweist. Gemäß der Ausführungsform von Fig. 1 berücksichtigt die Auswerteeinheit 24 diese Richtungsabhängigkeit, um die Richtungsinformation der Empfangselektrode 20 zu errechnen.

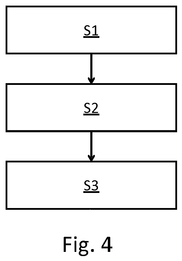

Es kann vorteilhaft sein, zuerst die Richtungsinformation (Winkel ϕ und optional eine Information über die Körperseite), und erst danach in einem separaten Schritt die Entfernung r zu ermitteln. Ein entsprechender Algorithmus ist in Fig. 4 skizziert. Der Algorithmus von Fig. 4 umfasst die folgenden Schritte:
S1: Die Auswerteeinheit 24 empfängt das elektrische Signal von der Empfangselektrode 20. Optional sind auf dem Körper des Patienten Referenzelektroden (EKG-Elektroden) A mit bekannten Positionen angebracht, wie in Fig. 1 und 4 dargestellt, und die Auswerteeinheit 24 empfängt elektrische Signale (EKG-Kanalsignale) von den EKG-Elektroden. Die EKG-Kanalsignale können relative Spannungsverläufe zwischen jeweiligen EKG-Elektrodenpaaren sein, oder deren Spannung gegen eine Referenzspannung. Die Referenzspannung kann eine Erdspannung sein, etwa das sogenannte Wilson central terminal: Dieses wird über eine Zusammenschaltung aller EKG-Elektroden gebildet und dient als Quasi-Erdpotential. Zusätzlich oder alternativ kann auch die Spannung (oder eine andere elektrische Kenngröße, etwa der Strom) zwischen der Empfangselektrode 20 und den EKG-Elektroden und/oder der Referenzspannung gemessen werden.
S2: Die Auswerteeinheit 24 vergleicht das von der Empfangselektrode 20 empfangene elektrische Signal mit den empfangenen EKG-Kanalsignalen im Hinblick auf eine Richtungscharakteristik, um die Richtungsinformation zu ermitteln. Dies kann beispielsweise dadurch geschehen, dass die Auswerteeinheit 24 ein hinsichtlich der Richtungscharakteristik am meisten übereinstimmendes EKG-Kanalsignal von den EKG-Elektroden A ermittelt und die Richtung der entsprechenden EKG-Elektrode als die ermittelte Richtungsinformation speichert.

Weitere Einzelheiten können wie bereits oben allgemein für das Errechnen der Position beschrieben umgesetzt werden, in diesem Schritt auf die Richtung anstelle der Position bezogen. So kann die Auswerteeinheit 24 beispielsweise eine Richtungsinformation basierend auf den Richtungen mehrerer EKG-Kanalelektroden relativ zum Herz ermitteln, indem etwa der jeweilige Übereinstimmungsgrad des EKG-Signals der Empfangselektrode 20 mit jedem der EKG-Kanalsignale ermittelt wird, und sodann eine Richtung der Empfangselektrode wie bereits oben beschrieben als Funktion der Übereinstimmungsgrade ermittelt wird..

Vor oder bei jedem Vergleich kann das an der Empfangselektrode 20 (und optional auch der an den EKG-Kanalelektroden) gemessene Signal (Spannungsverlauf) transformiert werden, um die Abhängigkeit des Signals vom Abstand zu reduzieren; etwa durch eine Skalierung des Signals. Damit wird sichergestellt, dass die Signale maßgeblich im Hinblick auf ihre Richtungsabhängigkeit verglichen werden. Die Skalierung kann durch eine Multiplikation des Spannungsverlaufs derart realisiert werden, dass das Maximum der Absolutwerte des an der Empfangselektrode 20 gemessenen Spannungsverlaufs gleich ist dem entsprechenden Maximums der an den EKG-Kanalelektroden gemessenen bzw. modellierten Spannungsverläufe. Diese Skalierung erlaubt eine verbesserte Vergleichbarkeit im Hinblick auf die Richtung, denn für die Richtungskomponente ist nur die Form, nicht die Skalierung relevant.

Gemäß einer weiteren Variante kann die Auswerteeinheit 24 auch Kanalinformationen weiterer Kanäle aus den empfangenen EKG-Kanalsignalen errechnen, auch wenn für die weiteren Kanäle nicht zwingend eine EKG-Elektrode vorhanden ist, und sodann wie oben beschrieben verfahren. Diese Berechnung kann wie oben beschrieben erfolgen, etwa durch ein Modell, welches ein richtungsabhängiges EKG-Modellsignal angibt und dessen Modellparameter, etwa durch einen best-fit-Algorithmus, aus den gemessenen EKG-Kanalsignalen ermittelt werden.

Gemäß einer weiteren Alternative kann auf einige oder alle der EKG-Referenzelektroden A auch verzichtet werden und die Richtungsinformation unter Verwendung eines Modells für das EKG-Signal (siehe oben) errechnet werden..

Die Richtungscharakteristik ist vorzugsweise eine Charakteristik des EKG-Signals, die stark von der Richtung bzw. von dem EKG-Kanal abhängt, aber die eine wesentlich weniger ausgeprägte Abhängigkeit vom Abstand aufweist. Die Richtungscharakteristik kann beispielsweise eins oder mehrere der Folgenden sein: ein Vorzeichen (etwa im Vergleich zur Nulllinie) von P-Welle, QRS-Komplex und/oder T-Welle; ein relatives Verhältnis der Maxima von P-Welle, QRS-Komplex und/oder T-Welle zueinander; ein relatives Verhältnis der von P-Welle, QRS-Komplex und/oder T-Welle eingeschlossenen Fläche (Integral ihrer Differenz zur Nulllinie, bzw. des Absolutwerts derselben) zueinander.

S3: Die Auswerteeinheit 24 errechnet sodann einen Abstand r der Empfangselektrode 20 relativ zum Herzen H anhand eines Abstandsparameters des empfangenen EKG-Signals. Der Abstandsparameter kann beispielsweise die Amplitude, Laufzeit, Phasenverschiebung, eine andere vom Abstand r abhängige Größe des EKG-Signals, oder eine Kombination daraus umfassen. Ein bevorzugter Abstandsparameter ist eine (absolute) Amplitude des gemessenen Spannungsverlaufs (z.B. Maximalamplitude oder mittlere Amplitude des Spannungsverlaufs oder ausgewählter Teile davon). Auch andere Abstandsparameter, die eine starke Abhängigkeit vom Abstand aufweisen, können zusätzlich oder stattdessen herangezogen werden.

Die Auswerteeinheit 24 kann den Abstand r durch Vergleich des Abstandsparameters mit dem Abstandsparameter des von den EKG-Referenzelektroden A empfangenen EKG-Signals ermitteln.

Alternativ oder zusätzlich kann die Auswerteeinheit 24 den Abstand r auch aufgrund einer Rechenvorschrift, etwa ausgehend von einem theoretischen Modell, ermitteln (wie oben bereits allgemein für das Ermitteln der Position beschrieben; hier also mit dem Abstand r als die Position).

Alternativ oder zusätzlich kann die Auswerteeinheit 24 den Abstand r auch separat ausgehend von empirischen Messwerten ermitteln, etwa durch Leitwert-, Impedanz- oder ähnliche Messungen etwa gegenüber einer oder mehreren der stationären Elektroden.

Gemäß einer Ausführungsform wird eine Amplitude (etwa der absoluten Maximalwert) des von der Empfangselektrode gemessenen Spannungsverlaufs als Abstandsparameter verwendet, und die Auswerteeinheit berechnet den Abstand mittels einer Abstandsfunktion in Abhängigkeit des Abstandsparameters und optional des bereits in Schritt S2 ermittelten Winkels. Die Abstandsfunktion kann beispielsweise mittels einer Dipolgleichung den Abstand bestimmen, der ein auf den Winkel projiziertes Dipolfeld mit der gemessenen Amplitude (Abstandsparameter) erzeugt. Zur Skalierung des modellierten Dipolfelds können die Abstandsparameter des von einer oder mehreren der EKG-Referenzelektroden A empfangenen EKG-Signals verwendet werden.

Zur Bestimmung des modellierten Feldes kann die Abstandsfunktion ein homogenes Medium annehmen. Alternativ kann die Abstandsfunktion auch ein nicht-homogenes elektrisches Körpermodell beinhalten, welches für verschiedene Körperteile unterschiedliche Impedanzen bzw. Dielektrizitätskonstanten vorsieht (z.B. eine Dipolgleichung in einem nicht-homogenen Medium).

Ein einfaches Beispiel für ein solches nicht-homogenes Körpermodell sieht für verschiedene Körperteile verschiedene Impedanzen Zᵢₚ vor. Diese Impedanzen können vorbestimmt oder für den individuellen Patienten durch einen vorherigen Kalibrationsschritt bestimmt sein, etwa mittels Messung an EKG-Referenzelektroden.

Das Feld kann mittels einer Dipolgleichung modelliert werden. Alternativ kann auch ein komplexeres Herzmodells genutzt werden, welches z.B. auch höhere Dipolmomente (bis zum 2., 3., 4. oder noch höherem Dipolmoment) beinhaltet. Alternativ oder zusätzlich kann die Modellierung des Feldes auch durch eine Finite-Elemente-Methode oder andere numerische Verfahren berechnet werden, oder ein anderes Herzmodell benutzt werden.

In dem oben beschriebenen Ablauf basierend auf Fig. 4 wurde die Ortsinformation als Richtungsinformation und Entfernung parametrisiert, die nacheinander ermittelt wurden (Schritte S2 und S3). Auch andere Parametrisierungen oder Koordinatensysteme sind möglich. Vorzugsweise verwendet die Auswerteeinheit für die Positionsermittlung eine null-, ein- oder zweidimensionale Parametrisierung (Koordinatensystem, optional mit diskreten zusätzlichen Angaben), welche auf Positionen auf der Hautoberfläche des Patienten beschränkt ist. Eine diskrete Angabe ist eine Angabe über wenige (z.B. maximal 16, 32 oder 64) abzählbare Auswahlmöglichkeiten wie Ober- oder Unterseite, diskrete Richtungsangabe wie EKG-Kanalnummer oder Körperteilangabe. Als nulldimensionale Parametrisierung wird eine Parametrisierung angesehen, die lediglich aus einer oder mehrerer (maximal vier) solcher diskreten Angaben besteht, wie etwa eine Körperteilangabe (z.B. Unter- oder Oberschenkel usw.). Mit einer solchen Parametrisierung, welche auf Positionen auf der Hautoberfläche des Patienten beschränkt ist, werden die in Frage kommenden Positionen des medizinischen Geräts von Anfang an und ohne besonderen Rechenaufwand auf die sinnvollen Positionen beschränkt. Dies erlaubt eine effiziente und ressourcensparende Berechnung der Position, da unsinnige Positionen bereits von Anfang an nicht berücksichtigt werden. Gleichzeitig wird sichergestellt, dass die ermittelte Position auf der Körperoberfläche liegt.

Alternativ oder zusätzlich zu der oben beschriebenen Auswertung sind auch weitere Konfigurationen der Auswerteeinheit 24 möglich, um die Charakteristik des empfangenen elektrischen Signals für die Berechnung der Ortsinformation der Empfangselektrode 20 auszuwerten. Beispielsweise ist eine Auswertung mittels eines Maschinenlern-Algorithmus möglich. Hierzu ist die Auswerteeinheit mit einem Maschinenlern-Modul ausgestattet.

Für die Konfiguration des Maschinenlern-Moduls kann zunächst ein Anlern-Prozess mittels eines Anlern-Moduls erfolgen, bei dem eine Vielzahl von aufgenommenen elektrischen Signalen sowie die (mit einem bekannten Positionierverfahren ermittelte) zugehörige Position des mobilen medizinischen Geräts M als Lernmaterial (sog. ground truth) in das Anlern-Modul eingelesen werden. Mit diesen Daten führt das Anlern-Modul einen Maschinen-Lern-Schritt durch, etwa mittels überwachten Maschinenlernens (sog. supervised learning algorithmus). In dem Maschinen-Lern-Schritt ermittelt (lernt) das Anlern-Modul diejenigen Merkmale des elektrischen Signals aus den vorhandenen Signalen, sowie deren Kombination und Gewichtung, welche statistisch den Fehler zwischen gelernter Positionsvoraussage und korrespondierender zugehöriger Position (ground truth) minimieren (was beinhaltet, das Minimum unter gegebenen Bedingungen lediglich anzunähern). Das Ergebnis dieses Anlern-Prozesses ist eine erlernte Funktion, welche zu einem eingegebenen elektrischen Signal - mittels der ermittelten Merkmale und deren Kombination und Gewichtung - eine errechnete Position ausgibt. Optional kann das Anlern-Modul die erlernte Funktion auch für weitere Eingabeparameter (etwa den bereits beschriebenen Atemstatus oder andere Sensordaten) und/oder für weitere Ausgabeparameter (zusätzlich zur errechneten Position) erzeugen.

Das Anlern-Modul kann im Maschinenlern-Modul integriert sein oder separat vorgesehen sein. Bei separatem Anlern-Modul kann der Anlern-Schritt einmalig erfolgen (etwa bei der Entwicklung der Auswerteeinheit), und das Maschinenlern-Modul kann dann lediglich mit der resultierenden erlernten Funktion ausgestattet sein.

Bei integriertem Anlern-Modul ist ein Anlernen und/oder eine Feinkalibrierung mittels patientenspezifischer (ggf. zusätzlicher) Daten als Lernmaterial möglich.

Fig. 2 zeigt eine Ausführungsform einer erfindungsgemäßen Vorrichtung 1, die im Folgenden beschrieben wird. Ebenso wie in Fig. 1 ist auch in Fig. 2 eine Empfangselektrode 20 am medizinischen Gerät M angebracht. Anders als in Fig. 1 erfolgt die Positionsbestimmung jedoch mittels aktiv gesendeter Signale statt der Signale vom Herzen H. Genauer sind in der Ausführungsform von Fig. 2 mehrere Sendeelektroden 10 an der Körperoberfläche des Patienten P angebracht. Die Sendeelektroden 10 haben jeweils eine vordefinierte bzw. bekannte Positionen zueinander.

Die Sendeelektroden 10 sind mit einer Spannungsquelle 14 operativ verbunden. Bei der Spannungsquelle 14 kann statt der Spannung auch ein anderer Wert geregelt sein, etwa ein Strom; eine solche Stromquelle und dergleichen wird hierin auch als Spannungsquelle verstanden. Die Spannungsquelle 14 erzeugt elektrische Signale, die Sendeelektroden 10 leiten diese elektrischen Signale sodann an die Hautoberfläche des Patienten, von wo aus die elektrischen Signale entlang der Hautoberfläche des Patienten weiter propagieren. Hierbei beinhaltet "entlang der Hautoberfläche" auch die Propagation entlang weiterer, in Beziehung zur Haut stehender Strukturen wie Blutbahnen, und schließt die Propagation entlang weiterer Strukturen, die nicht in direkter Beziehung zur Haut stehen, nicht aus.

Der weitere Ablauf erfolgt dann analog zu der Ausführungsform in Fig. 1: Die an dem medizinischen Gerät M angebrachte Empfangselektrode 20 empfängt die von den Sendeelektroden 10 ausgesendeten elektrischen Signale (im Weiteren auch kollektiv als empfangenes Signal bezeichnet); und die Auswerteeinheit 24 wertet das empfangene Signal dann aus, indem es daraus eine Ortsinformation der Empfangselektrode 20 relativ zu den Sendeelektroden 10 errechnet. Ein Unterschied ist jedoch, dass das elektrische Signal nicht vom Herzen H, sondern von einer zur Vorrichtung gehörigen Spannungsquelle 14 erzeugt und von einer oder mehreren Sendeelektroden 10 gesendet wird. Dadurch ist sichergestellt, dass das gesendete Signal eine bekannte und angepasste Signalform hat, was eine noch höhere Genauigkeit bei der Positionsbestimmung ermöglicht.

Dadurch, dass die Signalform des gesendeten Signals bereits bekannt ist, kann auch einfacher auf Referenzelektroden verzichtet werden, und das von der Empfangselektrode 20 empfangene Signal direkt mittels eines Modells ausgewertet werden.

In der Ausführung von Fig. 2 sind mehrere Sendeelektroden 10 dargestellt. Die Spannungsquelle 14 kann eingerichtet sein, um für jede der Sendeelektroden 10 ein jeweils eigenes, von den übrigen elektrischen Signalen unterscheidbares elektrisches Signal zu erzeugen. Entsprechend kann die Spannungsquelle 14 für jede der Sendeelektroden 10 einen jeweils eigenen Spannungserzeuger oder -modulator aufweisen, der entweder mit den weiteren Spannungserzeugern in einem gemeinsamen Gehäuse untergebracht ist, oder der dezentral angeordnet ist, etwa bei der jeweils zugehörigen Sendeelektrode 10. Die elektrischen Signale können sich etwa im Hinblick auf ihre Frequenz und/oder in Bezug auf ihre zeitliche Taktung unterscheiden, etwa durch zeitliche Versetzung gepulster Signale, oder durch ein beliebiges anderes Modulationsverfahren voneinander unterscheidbar sein und vorzugsweise orthogonal zueinander sein. Wiederum soll hier der Begriff "Spannungsquelle" usw. auch die Erzeugung einer anderen elektrischen Regelungsgröße wie etwa eines Stroms umfassen.

Die Auswerteeinheit 24 kann entsprechend ausgestattet sein, um die Signale von jeder der Sendeelektroden 10 wieder voneinander zu trennen und etwa einem jeweils eigenen Kanal zuzuordnen. Die Auswerteeinheit wertet dann für jeden der Kanäle eine jeweilige Charakteristik des empfangenen elektrischen Signals aus und errechnet anhand dieser Charakteristiken eine Position der Empfangselektrode 20 (und damit des mobilen medizinischen Geräts M) relativ zu den Signalquellen 10.

In einer Ausführungsform ermittelt die Auswerteeinheit beispielsweise die Distanzen der Empfangselektrode 20 relativ zu den jeweiligen Sendeelektroden 10, und bestimmt die Position der Empfangselektrode 20 über Triangulation aus den berechneten Distanzen. Hierfür genügt es, wenn die Positionen der Sendeelektroden 10 bekannt sind und wenn die Auswerteeinheit 24 eingerichtet ist, die Signale von den verschiedenen Sendeelektroden 10 voneinander zu unterscheiden (wie oben geschildert). Es ist außerdem nicht erforderlich, dass die Sendeelektroden 10 ein richtungsabhängiges Signal aussenden; vielmehr sind Quasi-Punktquellen als Sendeelektroden 10 ausreichend. Vorzugsweise sind in dieser Ausführungsform mindestens 3 oder sogar mindestens 4 Sendeelektroden 10 vorhanden.

Gemäß einer weiteren Variation sind die Sendeelektroden 10 ausgestattet, um ein richtungsabhängiges elektrisches Signal zu erzeugen, also ein Signal, das signifikante Dipol- und optional höhere Momente hat. Ein solches richtungsabhängiges Signal ist etwa durch eine als Elektrodenarray gestaltete und angesteuerte oder durch eine nicht-kreissymmetrisch geformte Sendeelektrode 10 möglich.

In diesem Fall genügen zwei Sendeelektroden oder sogar nur eine Sendeelektrode 10. Die Positionsbestimmung kann dann auf analoge Weise wie in der in Fig. 1 gezeigten passiven Variante erfolgen: Die Auswerteeinheit 24 errechnet dann anhand der Richtungsabhängigkeit des elektrischen Signals eine Richtungsinformation der Empfangselektrode 20 relativ zu den Sendeelektroden bzw. der Sendeelektrode 10, etwa auf Basis eines Modells wie bereits oben beschrieben.

Auch Mischformen sind möglich, etwa eine Elektrode, die sowohl ein richtungsunabhängiges Signal (zur Abstandsbestimmung) als auch ein richtungsabhängiges Signal (zur Richtungsbestimmung) zu erzeugen vermag, etwa abwechselnd; oder mehrere Elektroden, von denen einige richtungsabhängige Signale und einige andere richtungsunabhängige Signale erzeugen.

Gemäß einem weiteren Aspekt kann auch die Geräteelektrode G (Empfangselektrode 20) zum Empfangen einer Richtungsinformation des empfangenen Signals gestaltet sein, also etwa nicht-kreissymmetrisch geformt oder als Elektrodenarray gestaltet sein. Diese Richtungsinformation kann dann ebenfalls für die Errechnung der Position genutzt werden.

Die Sendeelektroden 10 können zugleich als EKG-Elektroden A eines EKG-Gerätes ausgeführt sein. Falls eine EKG-Messung ohnehin vorgesehen ist, ist in diesem Fall der apparative Mehraufwand für die Positionsbestimmung verringert und vor allem der zusätzlich zur ohnehin stattfindenden EKG-Messung notwendige Vorbereitungsaufwand am Körper des Patienten P vernachlässigbar klein.

Fig. 3 zeigt eine weitere Ausführungsform einer erfindungsgemäßen Vorrichtung 1. Hier sind gegenüber Fig. 2 Sender und Empfänger vertauscht: Nun ist eine Sendeelektrode 10 als die Geräteelektrode G am medizinischen Gerät M angebracht, und mehrere Empfangselektroden 20 sind an der Körperoberfläche des Patienten P angebracht.

Wie in Fig. 2 ist die Sendeelektrode 10 (nun aber als die Geräteelektrode G) mit einer Spannungsquelle 14 operativ verbunden, um das von der Spannungsquelle 14 erzeugte elektrische Signal an die Hautoberfläche des Patienten zu leiten. Weiter ist die Empfangselektrode 20 (optional mehrere Elektroden 20, an definierten Positionen an der Körperoberfläche des Patienten P angebracht) mit einer Auswerteeinheit 24 zum Auswerten des von der Empfangselektrode 20 empfangenen Signals verbunden.

Analog zu der Beschreibung von Fig. 2 ist die Auswerteeinheit 24 auch hier imstande, anhand einer Charakteristik des empfangenen elektrischen Signals eine Ortsinformation der Empfangselektrode 20 (die nun das Referenzsystem darstellt) relativ zu der Signalquelle 10 (deren Position nun mit der des mobilen medizinischen Geräts M korreliert ist) zu errechnen, und aus dieser Ortsinformation die Position des mobilen medizinischen Geräts M zu errechnen. Dies kann analog zu Fig. 2 im Fall mehrerer Empfangselektroden 20 durch Triangulation erfolgen, oder auf eine andere in Bezug auf Fig. 2 diskutierte Weise.

In der Ausführung von Fig. 3 sind mehrere Empfangselektroden 20 dargestellt; eine Ausführung mit nur einer Empfangselektrode 20 ist jedoch ebenfalls möglich, analog wie bereits oben zu Fig. 2 beschrieben. Bevorzugt ist (sind) die Empfangselektrode(n) 20 in EKG-Elektrode(n) integriert, so dass keine oder weniger gesonderte Elektroden am Patienten angebracht werden müssen.

Die Ausführungsformen von Fig. 2 und 3 haben den besonderen Vorteil, dass hier ein kontrolliertes, von der Signalquelle 14 erzeugtes Signal zur Positionierung genutzt wird. Dadurch ist eine besonders präzise Positionierung möglich. Die Ausführungsform von Fig. 2 hat den besonderen Vorteil, dass das Signal an vorbekannten Positionen dem Körper des Patienten zugeführt wird (nämlich an den Referenzelektroden bzw. EKG-Ableitungen A1-A4), und/oder dass eine Leistungsaufnahme an den Körper im Bereich des mobilen medizinischen Geräts M begrenzt wird.

Das medizinische Gerät wurde hierin beispielhaft als ein Ultraschall-Gerät beschrieben, es kann aber auch ein anderes Gerät sein, das bei Benutzung an der Hautoberfläche des Patienten zu positionieren ist. Bei einer solchen Benutzung bzw. Positionierung des mobilen medizinischen Geräts M stellt die Geräteelektrode G einen elektrischen Kontakt mit der Hautoberfläche des Patienten P her und funktioniert als Empfangselektrode 20 zum Empfangen des EKG-Signals.

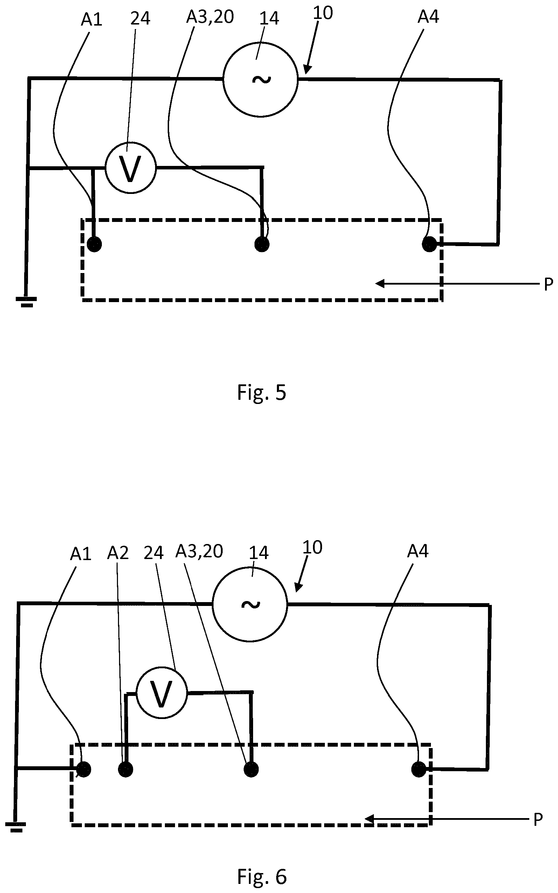

Fig. 5 zeigt eine Ausführungsform zur aktiven Positionsbestimmung mit einer Empfangselektrode am mobilen medizinischen Gerät.

An der Oberfläche des Patientenkörpers wurden EKG-Elektroden (Ableitungen) A1, ..., A4 in regelmäßigem Abstand (ca. 1-2 cm von Mittelpunkt zu Mittelpunkt) angebracht. Zwischen den äußersten EKG-Elektroden A1 und A4 wird mittels einer Spannungsquelle 14 eine Wechselspannung angelegt, wobei die Elektrode A1 geerdet ist. Der hierzu vorgesehene Schaltkreis einschließlich der EKG-Elektroden A1 und A4, und insbesondere die Elektrode A4, wird hierin auch als die Signalquelle 10 bezeichnet.

Eine weitere zwischen den Elektroden A1 und A4 liegende Elektrode A3 wird als die Empfangselektrode 20 verwendet. Mittels eines zur Auswerteeinheit 24 gehörenden Multimeters oder Oszilloskops werden die an dieser Elektrode anliegenden Spannungen (relativ zur Erde bzw. zur Elektrode A1 der Signalquelle 10) gemessen. Der Spannungsabfall entlang des Patientenkörpers P ist linear vom Abstand der Empfangselektrode 20 von der EKG-Elektrode A1 abhängig und erlaubt eine Positionsbestimmung der Empfangselektrode 20 mit Genauigkeiten deutlich feiner als 1 cm.

Die Ausführungsform von Fig. 6 entspricht der von Fig. 5, und die Beschreibung von Fig. 5 gilt auch hierfür, mit der folgenden Modifikation: In der Ausführungsform von Fig. 6 misst die Auswerteeinheit 24 die an der Empfangselektrode 20 anliegende Spannung relativ zu einer von der Sendeelektrode verschiedenen Referenzelektrode, hier relativ zur EKG-Elektrode A2. Die Referenzelektrode A2 ist somit eine nicht geerdete Floating-Elektrode.

Auch in der Ausführungsform von Fig. 6 ist der Spannungsabfall entlang des Patientenkörpers P linear vom Abstand der Empfangselektrode 20 von der EKG-Elektrode A2 abhängig und erlaubt eine Positionsbestimmung der Empfangselektrode 20 mit Genauigkeiten deutlich feiner als 1 cm.

Die Position der Referenzelektrode A2 relativ zur Signalquelle (z.B. zur Elektrode A1) ist vorbekannt. Daher wird durch eine Bestimmung der Relativposition gegenüber der Referenzelektrode A2 automatisch auch die Position relativ zur Signalquelle 10 bestimmt bzw. errechnet. Ein Vorteil der in Fig. 6 dargestellten Verschaltung liegt darin, dass die gemessenen Spannungen betragsmäßig niedriger sind als in Fig. 5 und darin, dass der positionsabhängige Spannungsgradient daher relativ zur gemessenen Spannung größer ist.

Gemäß einem allgemeinen und von der beschriebenen Ausführungsform unabhängigen Aspekt ist das von der Auswerteeinheit 24 empfangene elektrische Signal ein zwischen der Empfangselektrode 20 und einer Floating-Referenzelektrode anliegendes Signal, wobei die Floating-Referenzelektrode von der Sendeelektrode verschieden ist und/oder wobei kein externes Spannungssignal und/oder kein Erdkontakt an der Floating-Referenzelektrode anliegt, wobei vorzugsweise die Floating-Referenzelektrode elektrisch lediglich an den Patientenkörper gekoppelt ist.

Im Folgenden wird ein weiterer Aspekt der Erfindung beschrieben, der besonders relevant für den Fall ist, dass das mobile medizinische Gerät eine Ultraschallsonde für Ultraschall-Bildgebung beinhaltet. In diesem Fall ist es üblich, ein Ultraschallmedium auf die Hautoberfläche des Patienten zu applizieren, um einen möglichst lückenlosen Schallübergang zu gewährleisten. Gemäß einem Aspekt der Erfindung wird das Ultraschallmedium verwendet, um zumindest einen Teil (vorzugsweise einen überwiegenden Teil, d.h. mehr als 50% der Intensität) des entlang der Hautoberfläche des Patienten propagierenden elektrischen Signals zu tragen. Gemäß einem Aspekt hat das Ultraschallmedium eine definierte elektrische Impedanz bzw. Leitfähigkeit. Gemäß einem Aspekt ist die Leitfähigkeit betragsmäßig kleiner als 50 · 10⁻³ S/m (also kleiner als die Leitfähigkeit von Wasser), vorzugsweise kleiner als 30 · 10⁻³ S/m oder sogar kleiner als 10 · 10⁻³ S/m. Dadurch wird ein ausreichender Signalabfall auf für die Ortsbestimmung relevanten Längenskalen sichergestellt. Übliche Ultraschallmedien haben dagegen eine höhere Leitfähigkeit als Wasser und erlauben daher eine Ortsbestimmung nur mit niedrigerer Genauigkeit. Das erfindungsgemäße Ultraschallmedium (z.B. Ultraschallgel) kann hergestellt werden, indem das üblicherweise bei der Herstellung verwendete Wasser ganz oder teilweise durch destilliertes Wasser ersetzt wird. Gemäß einem Aspekt ist zumindest die Geräteelektrode eingerichtet, um zum Senden oder Empfangen des elektrischen Signals elektrisch an das auf die Hautoberfläche des Patienten aufgetragene Ultraschallmedium zu koppeln. Gemäß einem Aspekt ist die Auswerteeinheit dazu eingerichtet, um die Ortsinformation unter Berücksichtigung der elektrischen Leitfähigkeit des Ultraschallmediums zu errechnen, etwa aufgrund einer voreingestellten Leitwert-Information oder aufgrund einer Kalibrierungsmessung.

Gemäß einem weiteren Aspekt kann an dem mobilen medizinischen Gerät eine Vielzahl von Geräte-Elektroden (Sende- oder vorzugsweise Empfangselektroden) angebracht sein. Gemäß einem Aspekt ist die Auswerteeinheit mit der Vielzahl von Geräte-Elektroden operativ verbunden, um aus zumindest einem für die Vielzahl von Geräte-Elektroden ermittelten Relativwert eine Orientierungsinformation des medizinischen Geräts zu ermitteln. In einer Variante kann die Auswerteeinheit ein zwischen Paaren der Geräte-Elektroden anliegendes elektrisches Signal, etwa eine Relativspannung, empfangen und daraus eine relative Lage der Geräte-Elektroden auswerten, um daraus die Orientierungsinformation zu ermitteln. In einer weiteren Variante kann die Auswerteeinheit für die Vielzahl der Geräte-Elektroden eine jeweilige Ortsinformation wie hierin beschrieben ermitteln, und aus der Vielzahl von Ortsinformationen die Orientierungsinformation ermitteln. Die Orientierungsinformation kann insbesondere eine lokale Verdrehung (Drehwinkel) auf der Hautoberfläche sein. Gemäß einem weiteren Aspekt kann die Auswerteeinheit auch ausgestattet sein, um das elektrische Signal und/oder die für die Vielzahl der Geräte-Elektroden ermittelten jeweiligen Ortsinformationen zu mitteln, um die Genauigkeit der Ortsbestimmung zu erhöhen.

Gemäß einem weiteren Aspekt weist das medizinische Gerät ein weiteres Positioniersystem auf. Das weitere Positioniersystem erlaubt es, mindestens eine Positioniersystem-Ortsinformation (z.B. ein Orientierungswinkel oder Positionskoordinate oder eine Kombination daraus) des medizinischen Geräts zu erfassen, zusätzlich zu der von der Auswerteeinheit 24 wie oben beschrieben ermittelten Ortsinformation (im Folgenden auch als Elektroden-Ortsinformation bezeichnet).

Das weitere Positioniersystem kann vorzugsweise ein referenzloses Positioniersystem sein, etwa ein Inertial-Positioniersystem oder ein bildbasiertes Positioniersystem. Insbesondere, wenn das medizinische Gerät M ein medizinisches Bildgebungsgerät wie ein Ultraschallgerät ist, kann das Positioniersystem die Positioniersystem-Ortsinformation basierend auf der von dem medizinischen Bildgebungsgerät generierten Bildinformation erfassen. Beispielsweise kann das medizinische Gerät ein beliebiges der im Folgenden Veröffentlichungen beschriebenen Positioniersysteme aufweisen. In diesen Beispielen ist das medizinische Gerät:
- Inside-out Tracking des Schallkopfes durch eine am medizinischen Gerät befestigte optische Kamera, wobei die Kamera vorzugsweise derart befestigt ist, um bei Betrieb auf die Hautoberfläche des Patienten gerichtet zu sein. Das medizinische Gerät ist vorzugsweise ein Ultraschall-Bildgebungsgerät. Ein derartiges Positioniersystem ist beispielsweise beschrieben in: Sun SY., Gilbertson M., Anthony B.W. (2014) Probe Localization for Freehand 3D Ultrasound by Tracking Skin Features. In: Golland P., Hata N., Barillot C., Hornegger J., Howe R. (eds) Medical Image Computing and Computer-Assisted Intervention - MICCAI 2014. MICCAI 2014. Lecture Notes in Computer Science, vol 8674. Springer, Cham;
- Inertialsensortracking durch einen am medizinischen Gerät befestigten Beschleunigungssensor, vorzugsweise ergänzt durch einen optischen Sensor. Ein derartiges Positioniersystem ist beispielsweise beschrieben in: P. J. Stolka, H. J. Kang, M. Choti and E. M. Boctor, "Multi-DoF probe trajectory reconstruction with local sensors for 2D-to-3D ultrasound," 2010 IEEE International Symposium on Biomedical Imaging: From Nano to Macro, Rotterdam, 2010, pp. 316-319.

- Wenn das medizinische Gerät ein Bildgebungsgerät, vorzugsweise ein Ultraschall-Bildgebungsgerät ist, kann das Positioniersystem eine Bilderkennungseinheit zum Tracking basierend auf den Bilddaten (z.B. Ultraschallbilddaten) umfassen. Ein derartiges Positioniersystem ist beispielsweise beschrieben in: Gao, Haitao, et al. "Wireless and sensorless 3D ultrasound imaging." Neurocomputing 195 (2016): 159-171.

Die Auswerteeinheit 24 kann mit dem weiteren Positioniersystem verbunden sein, um die Positioniersystem-Ortsinformation zu empfangen und mit der Elektroden-Ortsinformation zu verknüpfen. Beispielsweise kann die Auswerteeinheit 24 eine erste Koordinate (z.B. Ortskoordinaten für die Position des medizinischen Geräts M) aus der Elektroden-Ortsinformation übernehmen und eine zweite, zu den ersten Koordinate komplementäre (linear unabhängige) Koordinate (z.B. Winkelkoordinaten für eine Orientierung, insbesondere für einen Verkippwinkel des medizinischen Geräts M) aus der Positioniersystem-Ortsinformation übernehmen und beide Koordinaten zu einer kombinierten Positions- und Orientierungsinformation verknüpfen.

In einem weiteren Beispiel kann die Auswerteeinheit 24 eine erste Koordinate aus der Elektroden-Ortsinformation mit einer zweiten, mit der ersten Koordinate zumindest teilweise übereinstimmenden bzw. redundanten (linear abhängigen) Koordinate aus der Positioniersystem-Ortsinformation verknüpfen (z.B. jeweils Ortskoordinaten für die Position des medizinischen Geräts M miteinander verknüpfen). Diese Verknüpfung kann beispielsweise eine (optional gewichtete) Mittelwertbildung zur Verbesserung der Genauigkeit umfassen. Die Verknüpfung kann auch eine Bestimmung der Abweichung umfassen, um einen Fehlerwert zu ermitteln oder um ein Warnsignal bei zu hoher Abweichung zu generieren.

Der Positionier-Ortsinformation kann das gleiche Referenzsystem (Basis des Koordinatensystems) oder ein anderes Referenzsystem als der Elektroden-Ortsinformation zugrunde liegen. Im zweiten Fall ist das Auswertesystem vorzugsweise eingerichtet, um die Positionier-Ortsinformation und/oder die Elektroden-Ortsinformation in einem gemeinsamen Referenzsystem bzw. Koordinatensystem darzustellen.

Gemäß einem weiteren Aspekt umfasst die Vorrichtung weiter ein Elektroden-Erfassungssystem zur Erfassung einer Position zumindest einiger der hierin beschriebenen Elektroden, insbesondere der EKG-Ableitung(en) in einem Referenzkoordinatensystem. Die Position der Elektroden kann dabei anhand der vollständigen Elektroden, anhand eines Teils der Elektroden oder anhand einer Elektrodenmarkierung (z.B. eines markierten Pflasters), welche zusätzlich zu der oder anstelle der Elektroden am Körper des Patienten angebracht worden ist, erfasst werden.

Die Auswerteeinheit kann ausgestattet sein, um aus der errechneten Ortsinformation der Empfangselektrode relativ zu der Signalquelle - und somit in Ausführungsformen aus der errechneten Ortsinformation des mobilen medizinischen Geräts relativ zu den EKG-Ableitungen - sowie aus der erfassten Position der EKG-Ableitungen eine Ortsinformation des mobilen medizinischen Geräts in dem Referenzkoordinatensystem zu errechnen. Das Elektroden-Erfassungssystem kann durch jedes beliebige bekannte Positionsbestimmungssystem implementiert sein, z.B. durch ein optisches Positionserfassungssystem.

Gemäß einem Aspekt umfasst die Vorrichtung weiter ein (stationäres) Bildgebungssystem, beispielsweise ein Magnetresonanztomographie-System oder ein Nuklear-Bildgebungssystems wie z.B. ein CT-Bildgebungssystem. In diesem Fall kann das Elektroden-Erfassungssystem durch ein Bilderkennungssystem realisiert sein, welches eingerichtet ist, um eine Position der Elektrode(n) und/oder einer Elektrodenmarkierung in einem CT-Bild in Bildkoordinaten des CT-Bildgebungssystems zu errechnen.

Gemäß einem Aspekt kann das oben beschriebene Referenzkoordinatensystem daher das Koordinatensystem des oben beschriebenen externen Bildgebungssystems sein (worunter jedes Koordinatensystem, für welches eine Umrechnung in bzw. aus Bildkoordinaten des externen Bildgebungssystems zur Verfügung steht). Dadurch wird es ermöglicht, mittels der Auswerteeinheit eine Ortsinformation des mobilen medizinischen Geräts in dem Koordinatensystem des Bildgebungssystems zu errechnen. Gemäß einem bevorzugten Aspekt umfasst die Vorrichtung in diesem Fall ein Modul zur Darstellung des mobilen medizinischen Geräts in einem von dem Bildgebungssystem errechneten Bild. Gemäß einem weiteren bevorzugten Aspekt umfasst das mobile medizinische Gerät einen Bildsensor zum Erstellen eines mobilen Bildes (z.B. Ultraschall-Bild), und die Vorrichtung umfasst ein Modul zur Darstellung des mobilen Bildes in dem Koordinatensystem des Bildgebungssystems. Nach einem besonders bevorzugten Aspekt umfasst die Vorrichtung ein Fusions-Modul zur überlagerten Darstellung des mobilen Bildes (z.B. Ultraschall-Bild) gemeinsam mit dem von dem stationären Bildgebungssystem errechneten Bild (z.B. CT-Bild).

Die hierin für die Vorrichtung beschriebenen Aspekte sind gemäß einem Aspekt insbesondere auch in dem hierin beschriebenen Verfahren einsetzbar. Umgekehrt ist die hierin beschriebene Vorrichtung gemäß einem Aspekt insbesondere auch dazu eingerichtet, jeden beliebigen der hierin beschriebenen Verfahrensschritte durchzuführen.

Gemäß einem allgemeinen Aspekt wird ein EKG-Signal oder ein von EKG-Ableitungen A1-A4 gesendetes oder empfangenes Signal zur Positionsbestimmung des medizinischen Gerätes genutzt. Dabei handelt es sich um ein elektrisches Signal, welches am Körper des Patienten abgegeben wird und entlang der Hautoberfläche propagiert. Als weitere (Sende- oder Empfangs-)Elektrode wird eine am medizinischen Gerät angebrachte Elektrode genutzt. Damit wird der für die Positionsbestimmung vorzusehende zusätzliche apparative und einrichtungsmäßige Aufwand minimiert.

Im Gegensatz hierzu wird etwa bei US 5,795,298 lediglich eine gemeinsame Hardware für EKG-Messung und Tracking vorgeschlagen. Das Tracking selbst erfolgt jedoch auf die vorbekannte Weise mittels elektromagnetischen Feldern im Raum.

## Patentansprüche

1. Vorrichtung (1) zum Erfassen der Position eines mobilen medizinischen Gerätes (M) an der Hautoberfläche eines menschlichen oder tierischen Patienten (P) mittels eines von einer Signalquelle (10) am Körper des Patienten abgegebenen und entlang der Hautoberfläche propagierenden elektrischen Signals, die Vorrichtung umfassend:
- das mobile medizinische Gerät, wobei das mobile medizinische Gerät ein medizinisches Bildgebungsgerät ist;
- eine Empfangselektrode (20) zum Empfangen des elektrischen Signals;
- eine mit der Empfangselektrode (20) operativ verbundene Auswerteeinheit (24) zum Auswerten des empfangenen elektrischen Signals,
- zumindest eine Spannungsquelle (14) zur Erzeugung des elektrischen Signals und eine mit der Spannungsquelle verbundene Sendeelektrode zum Leiten des elektrischen Signals an die Hautoberfläche des Patienten, so dass die Sendeelektrode die Signalquelle (10) bildet und wobei die Auswerteeinheit (24) konfiguriert ist, um anhand einer Charakteristik des empfangenen elektrischen Signals eine Ortsinformation der Empfangselektrode (20) relativ zu der Signalquelle (10) zu errechnen, und um aus der Ortsinformation eine Position des mobilen medizinischen Geräts (M) zu erhalten, **dadurch gekennzeichnet, dass** die Empfangselektrode oder die Signalquelle als Geräteelektrode an dem mobilen medizinischen Gerät angebracht ist, um einen elektrischen Kontakt mit der Hautoberfläche herzustellen, wenn das mobile medizinische Gerät an der Hautoberfläche positioniert ist.

2. Vorrichtung nach einem beliebigen der vorangehenden Ansprüche, wobei die Sendeelektrode zum Absenden eines richtungsabhängigen elektrischen Signals an die Hautoberfläche des Patienten ausgestaltet ist, und die Auswerteeinheit (24) konfiguriert ist, um anhand der Richtungsabhängigkeit des elektrischen Signals eine Richtungsinformation der Empfangselektrode (20) relativ zu der Signalquelle (10) zu errechnen.

3. Vorrichtung nach einem beliebigen der vorangehenden Ansprüche, wobei die Empfangselektrode (20) die Geräteelektrode (G) ist,
wobei die Sendeelektrode (10) vorzugsweise durch eine EKG-Ableitung (A1-A4) gebildet ist.

4. Vorrichtung nach einem beliebigen der beiden vorangehenden Ansprüche, umfassend eine Vielzahl von Sendeelektroden (10),
wobei vorzugsweise die Empfangselektrode (20) zum Empfangen eines jeweiligen elektrischen Signals von jeder der Vielzahl von Sendeelektroden (10) eingerichtet ist, und wobei die Auswerteeinheit (24) konfiguriert ist, um eine Ortsinformation der Empfangselektrode (20) anhand von einer Kombination von Charakteristiken der empfangenen elektrischen Signale zu errechnen.

5. Vorrichtung nach einem beliebigen der vorangehenden Ansprüche, wobei die Sendeelektrode (10) die Geräteelektrode (G) ist,
wobei die Empfangselektrode (20) vorzugsweise durch eine EKG-Ableitung (A1-A4) gebildet ist.

6. Vorrichtung nach dem vorangehenden Anspruch, umfassend eine Vielzahl von Empfangselektroden (20),
wobei vorzugsweise jede der Vielzahl der Empfangselektroden (20) zum Empfangen eines jeweiligen elektrischen Signals von der Sendeelektrode (10) eingerichtet ist, und wobei die Auswerteeinheit (24) konfiguriert ist, um eine Ortsinformation der Signalquelle (10) anhand von einer Kombination von Charakteristiken der empfangenen elektrischen Signale zu errechnen.

7. Vorrichtung nach einem beliebigen der vorangehenden Ansprüche, wobei (a) die Charakteristik des empfangenen elektrischen Signals zumindest einen Abstandsparameter ausgewählt aus Amplitude, Laufzeit, Frequenz, Phasenverschiebung des empfangenen elektrischen Signals umfasst, und wobei die Auswerteeinheit (24) konfiguriert ist, um anhand des Abstandsparameters einen Abstand der Empfangselektrode (20) relativ zu der Signalquelle (10) zu errechnen, und /oder
wobei (b) das elektrische Signal ein Wechselspannungssignal und/oder ein gepulstes Signal ist.

8. Vorrichtung nach einem beliebigen der vorangehenden Ansprüche, wobei die Auswerteeinheit mit einem Maschinenlern-Modul ausgestattet ist, um eine Ortsinformation der Empfangselektrode (20) relativ zu der Signalquelle (10) durch eine mittels Maschinenlernen erhaltenen Maschinenlern-Funktion anhand der Charakteristik des empfangenen elektrischen Signals zu errechnen.

9. Vorrichtung nach einem beliebigen der vorangehenden Ansprüche, weiter umfassend eine von der Sendeelektrode verschiedene Floating-Referenzelektrode, wobei das von der Auswerteeinheit (24) empfangene elektrische Signal ein zwischen der Empfangselektrode (20) und der Floating-Referenzelektrode anliegendes elektrisches Signal umfasst.

10. Vorrichtung nach einem beliebigen der vorangehenden Ansprüche, wobei die Geräteelektrode (G) in einer Vielzahl von Geräteelektroden (G) des mobilen medizinischen Geräts umfasst ist, und wobei die Auswerteeinheit mit der Vielzahl von Geräte-Elektroden (G) operativ verbunden ist, um aus zumindest einem für die Vielzahl von Geräte-Elektroden ermittelten Relativwert eine Orientierungsinformation des medizinischen Geräts zu ermitteln.

11. Vorrichtung nach einem beliebigen der vorangehenden Ansprüche, wobei die Vorrichtung ein zweites Positioniersystem aufweist, welches ausgestattet ist, mindestens eine Positioniersystem-Ortsinformation des medizinischen Geräts unabhängig von dem elektrischen Signal zu erfassen,
wobei die Auswerteeinheit (24) vorzugsweise mit dem zweiten Positioniersystem verbunden ist, um die Positioniersystem-Ortsinformation zu empfangen und mit der anhand der Charakteristik des empfangenen elektrischen Signals ermittelten Ortsinformation zu verknüpfen.

12. Vorrichtung nach einem beliebigen der vorangehenden Ansprüche, wobei die Vorrichtung ein Elektroden-Erfassungssystem zur Erfassung einer Position einer Referenzelektrode (A1, ..., A4) in einem Referenzkoordinatensystem, wobei die Referenzelektrode (A1, ..., A4) die von der Geräteelektrode (G) verschiedene Empfangselektrode (20) oder Signalquelle (10) umfasst, und
wobei die Auswerteeinheit eingerichtet ist, um aus der errechneten Ortsinformation der Empfangselektrode relativ zu der Signalquelle sowie aus der erfassten Position der Referenzelektrode (A1, ..., A4) eine Ortsinformation des mobilen medizinischen Geräts (M) in dem Referenzkoordinatensystem zu errechnen.

13. Vorrichtung nach einem beliebigen der vorangehenden Ansprüche, weiter umfassend ein stationäres Bildgebungssystem, wobei die Vorrichtung ein Modul zur Darstellung des mobilen Bildes in dem Koordinatensystem des stationären Bildgebungssystems umfasst.

14. Verfahren zum Erfassen der Position eines mobilen medizinischen Gerätes (M) an der Hautoberfläche eines menschlichen oder tierischen Patienten (P), wobei das mobile medizinische Gerät ein medizinisches Bildgebungsgerät ist, die Vorrichtung umfassend:
- eine Spannungsquelle (14) zur Erzeugung eines elektrischen Signals;
- eine mit der Spannungsquelle verbundene Sendeelektrode zum Leiten des elektrischen Signals an die Hautoberfläche des Patienten, so dass die Sendeelektrode eine Signalquelle (10) bildet;
- eine Empfangselektrode (20);
- eine mit der Empfangselektrode (20) operativ verbundene Auswerteeinheit (24), wobei
die Empfangselektrode (20) oder die Sendeelektrode als Geräteelektrode (G) an dem mobilen medizinischen Gerät (M) ausgebildet ist,
das Verfahren umfassend:
- Positionieren des mobilen medizinischen Geräts (M) an der Hautoberfläche des Patienten (P), so dass ein elektrischer Kontakt mit der Hautoberfläche des Patienten (P) hergestellt ist,
- Empfangen, durch die Empfangselektrode (20), eines elektrischen Signals, wobei das elektrische Signal von einer Signalquelle (10) am Körper des Patienten abgegeben wurde und von der Signalquelle (10) entlang der Hautoberfläche zu der die Empfangselektrode (20) propagiert ist;
- Auswerten, durch die Auswerteeinheit (24), des empfangenen elektrischen Signals, wobei die Auswerteeinheit (24) anhand einer Charakteristik des empfangenen elektrischen Signals eine Ortsinformation der Empfangselektrode (20) relativ zu der Signalquelle (10) errechnet, und aus der Ortsinformation eine Position des mobilen medizinischen Geräts (M) bestimmt.

15. Verwendung eines Ultraschallmediums für das Verfahren zum Erfassen der Position eines mobilen medizinischen Gerätes (M) nach Anspruch 14, wobei das mobile medizinische Gerät eine Ultraschallsonde umfasst, wobei das Ultraschallmedium im Bereich mindestens einer der Signalquelle (10) und der Empfangselektrode (20) auf die Hautoberfläche des Patienten appliziert wird,
wobei die Leitfähigkeit des Ultraschallmediums vorzugsweise betragsmäßig kleiner als die Leitfähigkeit von Wasser ist.

## Claims

1. An apparatus (1) for detecting the position of a mobile medical device (M) on the skin surface of a human or animal patient (P) by means of an electrical signal emitted by a signal source (10) on the body of the patient and propagating along the skin surface, the apparatus comprising:
- the mobile medical device, the mobile medical device being a medical imaging device;
- a receiving electrode (20) for receiving the electrical signal;
- an evaluation unit (24) that is operatively connected to the receiving electrode (20) for evaluating the received electrical signal,
- at least one voltage source (14) for generating the electrical signal and a transmitting electrode connected to the voltage source for conducting the electrical signal to the skin surface of the patient, such that the transmitting electrode forms the signal source (10), and
the evaluation unit (24) being configured to calculate a piece of location information of the receiving electrode (20) relative to the signal source (10) on the basis of a characteristic of the received electrical signal and to obtain a position of the mobile medical device (M) from the location information,
**characterized in that** the receiving electrode or signal source is attached to the mobile medical device in the form of a device electrode in order to establish electrical contact with the skin surface when the mobile medical device is positioned on the skin surface.

2. The apparatus according to any of the preceding claims, wherein the transmitting electrode is designed to transmit a direction-dependent electrical signal to the skin surface of the patient, and the evaluation unit (24) is configured to calculate a piece of direction information of the receiving electrode (20) relative to the signal source (10) on the basis of the directional dependence of the electrical signal.

3. The apparatus according to any of the preceding claims, wherein the receiving electrode (20) is the device electrode (G),
wherein the transmitting electrode (10) is preferably formed by an ECG lead (A1-A4).

4. The apparatus according to any of the two preceding claims, comprising a plurality of transmitting electrodes (10),
wherein preferably the receiving electrode (20) is constructed to receive a respective electrical signal from each of the plurality of transmitting electrodes (10), and wherein the evaluation unit (24) is configured to calculate a piece of location information of the receiving electrode (20) on the basis of a combination of characteristics of the received electrical signals.

5. The apparatus according to any of the preceding claims, wherein the transmitting electrode (10) is the device electrode (G),
wherein the receiving electrode (20) is preferably formed by an ECG lead (A1-A4).

6. The apparatus according to the preceding claim, comprising a plurality of receiving electrodes (20),
wherein preferably each of the plurality of receiving electrodes (20) is constructed to receive a respective electrical signal from the transmitting electrode (10), and wherein the evaluation unit (24) is configured to calculate a piece of location information of the signal source (10) on the basis of a combination of characteristics of the received electrical signals.

7. The apparatus according to any of the preceding claims, wherein (a) the characteristic of the received electrical signal comprises at least one distance parameter selected from the amplitude, propagation time, frequency, phase shift of the received electrical signal, and wherein the evaluation unit (24) is configured to calculate a distance of the receiving electrode (20) relative to the signal source (10) on the basis of the distance parameter, and/or
wherein (b) the electrical signal is an alternating-voltage signal and/or a pulsed signal.

8. The apparatus according to any of the preceding claims, wherein the evaluation unit is equipped with a machine-learning module in order to calculate a piece of location information of the receiving electrode (20) relative to the signal source (10) by means of a machine-learning function obtained by machine learning on the basis of the characteristic of the received electrical signal.

9. The apparatus according to any of the preceding claims, further comprising a floating reference electrode that is different from the transmitting electrode, wherein the electrical signal received by the evaluation unit (24) comprises an electrical signal applied between the receiving electrode (20) and the floating reference electrode.

10. The apparatus according to any of the preceding claims, wherein the device electrode (G) is contained in a plurality of device electrodes (G) of the mobile medical device, and wherein the evaluation unit is operatively connected to the plurality of device electrodes (G) in order to identify a piece of orientation information of the medical device from at least one relative value determined for the plurality of device electrodes.

11. The apparatus according to any of the preceding claims, wherein the apparatus includes a second positioning system which is equipped to detect at least one piece of positioning-system location information of the medical device independently of the electrical signal,
wherein the evaluation unit (24) is preferably connected to the second positioning system in order to receive the positioning-system location information and to link said information to the location information identified on the basis of the characteristic of the received electrical signal.

12. The apparatus according to any of the preceding claims, wherein the apparatus comprises an electrode detection system for detecting a position of a reference electrode (A1, ..., A4) in a reference coordinate system, wherein the reference electrode (A1, ..., A4) comprises the receiving electrode (20) or signal source (10) that is different from the device electrode (G), and
wherein the evaluation unit is constructed to calculate a piece of location information of the mobile medical device (M) in the reference coordinate system from the calculated location information of the receiving electrode relative to the signal source and from the detected position of the reference electrode (A1, ..., A4).

13. The apparatus according to any of the preceding claims, further comprising a stationary imaging system, wherein the apparatus comprises a module for displaying the mobile image in the coordinate system of the stationary imaging system.

14. A method for detecting the position of a mobile medical device (M) on the skin surface of a human or animal patient (P), wherein the mobile medical device is a medical imaging device, the apparatus comprising:
- a voltage source (14) for generating an electrical signal;
- a transmitting electrode connected to the voltage source for conducting the electrical signal to the skin surface of the patient, such that the transmitting electrode forms a signal source (10);
- a receiving electrode (20);
- an evaluation unit (24) that is operatively connected to the receiving electrode (20), wherein
the receiving electrode (20) or the transmitting electrode is formed as a device electrode (G) on the mobile medical device (M),
the method comprising:
- positioning the mobile medical device (M) on the skin surface of the patient (P) such that electrical contact with the skin surface of the patient (P) is established,
- receiving an electrical signal by means of the receiving electrode (20), wherein the electrical signal has been emitted by a signal source (10) on the body of the patient and is propagated from the signal source (10) along the skin surface to the receiving electrode (20);
- evaluating the received electrical signal by means of the evaluation unit (24),
wherein the evaluation unit (24) calculates a piece of location information of the receiving electrode (20) relative to the signal source (10) on the basis of a characteristic of the received electrical signal and determines a position of the mobile medical device (M) from the location information.

15. Use of an ultrasound medium for the method for detecting the position of a mobile medical device (M) according to claim 14, wherein the mobile medical device comprises an ultrasound probe, wherein the ultrasound medium is applied to the skin surface of the patient in the region of at least one out of the signal source (10) and the receiving electrode (20),
wherein the conductivity of the ultrasound medium is preferably lower than the conductivity of water.

## Revendications

1. Dispositif (1) pour détecter la position d'un appareil médical mobile (M) sur la surface de la peau d'un patient (P) humain ou animal au moyen d'un signal électrique délivré par une source de signal (10) sur le corps du patient et se propageant le long de la surface de la peau, le dispositif comprenant :
- l'appareil médical mobile, dans lequel l'appareil médical mobile est un appareil d'imagerie médicale ;
- une électrode de réception (20) pour recevoir le signal électrique ;
- une unité d'évaluation (24) reliée de manière opérationnelle à l'électrode de réception (20) pour évaluer le signal électrique reçu,
- au moins une source de tension (14) pour produire le signal électrique et une électrode d'émission reliée à la source de tension pour guider le signal électrique sur la surface de la peau du patient, de sorte que l'électrode d'émission forme la source de signal (10),
et dans lequel
l'unité d'évaluation (24) est configurée pour calculer sur la base d'une caractéristique du signal électrique reçu une information de lieu de l'électrode de réception (20) par rapport à la source de signal (10), et pour obtenir à partir de l'information de lieu une position de l'appareil médical mobile (M),
**caractérisé en ce que** l'électrode de réception ou la source de signal est montée en tant qu'électrode d'appareil sur l'appareil médical mobile, afin d'établir un contact électrique avec la surface de la peau, lorsque l'appareil médical mobile est positionné sur la surface de la peau.

2. Dispositif selon l'une quelconque des revendications précédentes, dans lequel l'électrode d'émission est conçue pour envoyer un signal électrique dépendant de la direction sur la surface de la peau du patient, et l'unité d'évaluation (24) est configurée pour calculer une information de direction de l'électrode de réception (20) par rapport à la source de signal (10) sur la base de la dépendance de direction du signal électrique.

3. Dispositif selon l'une quelconque des revendications précédentes, dans lequel l'électrode de réception (20) est l'électrode d'appareil (G),
dans lequel l'électrode d'émission (10) est formée de préférence par une dérivation d'ECG (A1-A4).

4. Dispositif selon l'une quelconque des deux revendications précédentes, comprenant une pluralité d'électrodes d'émission (10),
dans lequel de préférence l'électrode de réception (20) est agencée pour recevoir un signal électrique respectif de chacune de la pluralité d'électrodes d'émission (10), et
dans lequel l'unité d'évaluation (24) est configurée pour calculer une information de lieu de l'électrode de réception (20) sur la base d'une combinaison de caractéristiques des signaux électriques reçus.

5. Dispositif selon l'une quelconque des revendications précédentes, dans lequel l'électrode d'émission (10) est l'électrode d'appareil (G),
dans lequel l'électrode de réception (20) est formée de préférence par une dérivation d'ECG (A1-A4).

6. Dispositif selon la revendication précédente, comprenant une pluralité d'électrodes de réception (20),
dans lequel de préférence chacune de la pluralité des électrodes de réception (20) est agencée pour recevoir un signal électrique respectif de l'électrode d'émission (10), et dans lequel l'unité d'évaluation (24) est configurée pour calculer une information de lieu de la source de signal (10) sur la base d'une combinaison de caractéristiques des signaux électriques reçus.

7. Dispositif selon l'une quelconque des revendications précédentes, dans lequel (a) la caractéristique du signal électrique reçu comprend au moins un paramètre de distance choisi parmi l'amplitude, le temps de propagation, la fréquence, le décalage de phase du signal électrique reçu, et dans lequel l'unité d'évaluation (24) est configurée pour calculer une distance de l'électrode de réception (20) par rapport à la source de signal (10) sur la base du paramètre de distance, et/ou
dans lequel (b) le signal électrique est un signal de tension alternative et/ou un signal pulsé.

8. Dispositif selon l'une quelconque des revendications précédentes, dans lequel l'unité d'évaluation est équipée d'un module d'apprentissage machine, afin de calculer une information de lieu de l'électrode de réception (20) par rapport à la source de signal (10) par une fonction d'apprentissage machine obtenue au moyen d'un apprentissage machine sur la base de la caractéristique du signal électrique reçu.

9. Dispositif selon l'une quelconque des revendications précédentes, comprenant en outre une électrode de référence flottante différente de l'électrode d'émission, dans lequel le signal électrique reçu par l'unité d'évaluation (24) comprend un signal électrique s'appliquant entre l'électrode de réception (20) et l'électrode de référence flottante.

10. Dispositif selon l'une quelconque des revendications précédentes, dans lequel l'électrode d'appareil (G) est comprise dans une pluralité d'électrodes d'appareil (G) de l'appareil médical mobile, et dans lequel l'unité d'évaluation est reliée de manière opérationnelle à la pluralité d'électrodes d'appareil (G), afin de déterminer à partir d'au moins une valeur relative déterminée pour la pluralité d'électrodes d'appareil une information d'orientation de l'appareil médical.

11. Dispositif selon l'une quelconque des revendications précédentes, dans lequel le dispositif présente un deuxième système de positionnement, lequel est équipé pour détecter au moins une information de lieu de système de positionnement de l'appareil médical indépendamment du signal électrique,
dans lequel l'unité d'évaluation (24) est reliée de préférence au deuxième système de positionnement, afin de recevoir l'information de lieu de système de positionnement et de la combiner à l'information de lieu déterminée sur la base de la caractéristique du signal électrique reçu.

12. Dispositif selon l'une quelconque des revendications précédentes, dans lequel le dispositif comprend un système de détection d'électrode pour détecter une position d'une électrode de référence (A1, ..., A4) dans un système de coordonnées de référence, dans lequel l'électrode de référence (A1, ..., A4) comprend l'électrode de réception (20) ou source de signal (10) différente de l'électrode d'appareil (G), et
dans lequel l'unité d'évaluation est agencée pour calculer à partir de l'information de lieu calculée de l'électrode de réception par rapport à la source de signal ainsi qu'à partir de la position détectée de l'électrode de référence (A1, ..., A4) une information de lieu de l'appareil médical mobile (M) dans le système de coordonnées de référence.

13. Dispositif selon l'une quelconque des revendications précédentes, comprenant en outre un système d'imagerie fixe, dans lequel le dispositif comprend un module pour la représentation de l'image mobile dans le système de coordonnées du système d'imagerie fixe.

14. Procédé pour détecter la position d'un appareil médical mobile (M) sur la surface de la peau d'un patient (P) humain ou animal, dans lequel l'appareil médical mobile est un appareil d'imagerie médicale, le dispositif comprenant :
- une source de tension (14) pour produire un signal électrique ;
- une électrode d'émission reliée à la source de tension pour guider le signal électrique sur la surface de la peau du patient, de sorte que l'électrode d'émission forme une source de signal (10) ;
- une électrode de réception (20) ;
- une unité d'évaluation (24) reliée de manière opérationnelle à l'électrode de réception (20), dans lequel
l'électrode de réception (20) ou l'électrode d'émission est réalisée en tant qu'électrode d'appareil (G) sur l'appareil médical mobile (M),
le procédé comprenant les étapes suivantes :
- le positionnement de l'appareil médical mobile (M) sur la surface de la peau du patient (P), de sorte qu'un contact électrique avec la surface de la peau du patient (P) est établi,
- la réception, par l'électrode de réception (20), d'un signal électrique, dans lequel le signal électrique a été délivré à partir d'une source de signal (10) sur le corps du patient et est propagé à partir de la source de signal (10) le long de la surface de la peau vers l'électrode de réception (20) ;
- l'évaluation, par l'unité d'évaluation (24), du signal électrique reçu,
dans lequel l'unité d'évaluation (24) calcule sur la base d'une caractéristique du signal électrique reçu une information de lieu de l'électrode de réception (20) par rapport à la source de signal (10), et détermine à partir de l'information de lieu une position de l'appareil médical mobile (M).

15. Utilisation d'un milieu à ultrasons pour le procédé pour détecter la position d'un appareil médical mobile (M) selon la revendication 14, dans laquelle l'appareil médical mobile comprend une sonde à ultrasons, dans laquelle le milieu à ultrasons est appliqué dans la zone d'au moins une de la source de signal (10) et de l'électrode de réception (20) sur la surface de la peau du patient,
dans laquelle la conductivité du milieu à ultrasons est de préférence inférieure, en termes de valeur, à la conductivité de l'eau.
